# EUROPEAN PATENT APPLICATION

(11) **EP 3 410 113 A1**
(43) Date of publication of application: **05.12.2018**
(21) Application number: 18179146.8
(22) Date of filing: 02.05.2016
(51) Int. Cl.: G01N 33/28, E21B 47/10, G01V 5/12, G01N 23/203

(54) **METHODS AND SYSTEMS FOR LOGGING DATA RELATING TO FLUIDS WITHIN A CONDUIT**

(30) Priority: 30.04.2015 US 201562154955 P
(62) Divisional of application: 16725771.6
(71) Applicant: Visuray Intech Ltd. (BVI), Road Town, Tortola (VG); Schneiders, Servatius, 44791 Bochum (DE)
(72) Inventor: Kambiz, Safinya, Houston, TX 77056 (US); Spannuth, Melissa, 4008 Stavanger (NO); Gunn, Spencer, London, E14 7DG (GB)
(74) Representative: Schneiders & Behrendt PartmbB Rechtsanwälte - Patentanwälte

(57) **Abstract**

Methods and means for logging are also disclosed, and include at least a forward looking acquisition mode for use while the apparatus remains stationary; a plurality of acquisitions being performed while longitudinally moving the apparatus a predetermined distance through the conduit between acquisition operations; and a plurality of acquisitions being performed longitudinally while moving the apparatus a predetermined distance through the conduit.

## Description

The present invention relates generally to the fields of imaging and logging the contents and characteristics of wells, boreholes and hydrocarbon formations, and in a particular though nonlimiting embodiment to methods and means of measuring and characterizing fluids disposed within a borehole or pipe containing water, oil or gas, or a mixture thereof.

The oil and gas industry has traditionally classified reservoirs by structures subdivided into geological units or pressure compartments in order to characterize fluids in a formation. Typically, fluid samples from varying depths of the well would either be retrieved from the well or analyzed *in situ.*

Retrieved fluid samples typically must be maintained under similar pressures and temperatures as the sampling environment in order to ensure that the properties of the sample do not change due to partial release of contained gas pressure. Previously known *in situ* methods have therefore relied upon optical or ultrasonic means for characterizing the fluid while it is still captured in various depths of the well.

Despite the development and advancement of various methods for determining formation fluid properties based on acquiring formation fluid samples from inside the wellbore, there remains a need to provide techniques capable of determining the composition of fluids without altering their properties or physical location by the action of sample collection or interrogation.

There are currently several *in situ* methods for fluid recognition available to operators, *viz.:*
1. Optical fluid characterization;
2. Ultrasonic methods, including time of flight, particulate count and flow rate (Doppler);
3. Electromagnetic methods consisting of irradiation of fluids by gamma rays from radioactive isotopes or x-rays from Bremsstrahlung and detection of transmitted radiation for analysis of the attenuation characteristics of said fluid; and
4. Electromagnetic methods consisting of irradiation of fluids by gamma rays from radioactive isotopes or x-rays from Bremsstrahlung and detection of scattered radiation for analysis of the attenuation characteristics of said fluid.

The optical fluid characterization approach involves passing key wavelengths of light (*e.g.,* from infrared to ultraviolet) through the fluid to determine the attenuation coefficient of the fluid by analyzing the distribution of attenuation characteristics as a function of wavelength. By defining the characteristic distribution signature of the attenuation response to optical wavelengths of a fluid sample, the fluid under interrogation can be compared against a lookup table of known fluid attenuation characteristics and the most probable fluid type determined.

Ultrasonic methods involve transmission of pulses through the fluid between a transducer and a receiver over a predetermined distance, so that the time of flight can be measured and therefore the speed of sound within the fluid determined. Additionally, the ultrasonic properties of the fluid may be affected by the particulate content, thus the acoustic signature profile can be used to characterize the particulate content of the fluid. Other known means include using comparative time of flight paths in two directions through a moving fluid so that a Doppler shift can be measured and the speed of flow of the fluid determined.

Gamma and x-ray transmission methods consist of separating a sample of fluid from the main borehole and irradiating the sample with gamma or x-rays. A detector placed on the opposite side of the fluid compared to the radiation source then measures the amount and/or spectral energy distribution of the radiation that passes through the sample from said source. The radiation emitted by the source is attenuated in the fluid by an amount dependent upon the electron density profile of the fluid and the energy of the radiation. The resulting radiation transmitted through to the detector thus bears a signature of the composition of the intervening fluid. By comparing the amount and spectral energy distribution of the detected radiation against a database of known materials, the sample fluid can be identified.

Gamma and x-ray scattering methods consist of interrogating a fluid sample from the main borehole by irradiating the sample with gamma or x-rays, but without first isolating the sample from the borehole. A detector placed approximately adjacent to the radiation source then measures the amount and/or spectral energy distribution of the photons that scatter through the fluid from said source.

The radiation emitted by the source is scattered in the fluid by the amount related to the electron density profile of the fluid. Thus, the scattered radiation collected by the detector bears a signature of the composition of the borehole fluid. By comparing the amount and spectral energy distribution of the detected radiation against a database of known materials, the sample fluid can be identified.

The known prior art teaches a variety of techniques that use x-rays or other radiant energy to identify or obtain information about the fluid within the borehole of a water, oil or gas well, though none teach any method for interrogating the fluid in front of a tool while said tool is moving through the well as described and claimed later in the application. Such a method provides the benefit that the fluid has not been altered prior to interrogation by mixing, movement of interfaces or otherwise disturbed by passage of the tool.

For example:
US 4 980 642 A describes a method for determining the dielectric constant of a fluid within a borehole surrounding a drill pipe. The method uses radar to determine the conductivity of the fluid surrounding the drill pipe within a borehole.
US 4 994 671 A teaches a methods and means for analyzing the composition of formation fluids through optical spectroscopic methods, primarily by way of comparison between the emitted wavelength of a source light and the detected wavelength after passing through a fluid sample.
US 5 276 656 A describes a method for using ultrasonic techniques for fluid identification and evaluation in boreholes. The method teaches a temporal evaluation of the ultrasonic properties of a fluid based on the speed of sound within the fluid, with an aim of determining the volume of said fluid by calculating the rate of change of said fluid properties as a function of volume.
US 4 628 725 A describes a method for using ultrasonic techniques for fluid identification and evaluation in a tubular conduit, specifically those surrounding a drill string. The method teaches of a means to determine ultrasonic properties of a fluid based on the speed of sound within the fluid.
US 4 947 683 A describes an apparatus which employs a Doppler borehole fluid measuring scheme. A rotating ultrasonic head is described that would be capable of measuring the interfaces between fluid types within a borehole. The concept of measurements based on the Doppler effect measurements being possible by the flow of gas bubbles within the fluid is also discussed.
US 7 675 029 A provides an apparatus that permits the measurement of x-ray backscattered photons from any solid surface inside of a borehole that refers to two-dimensional imaging techniques. Teague *et al.* teaches use of spectroscopy and comparison with a materials database to determine the composition of the materials within the solid surface.
US 8 511 379 A describes a system and method for determining properties of a fluid based on the x-ray absorption of a fluid. The concept of transmission absorption with respect to a fluid is taught in addition to a multi-pixel array detector system which is employed to detect tracers within the fluid. However, it fails to teach of a system which combines all of the counts of each pixel such that the overall statistical noise can be reduced, thereby improving the quality of the signal.
US 7 807 962 A describes a system and method for determining properties of a fluid based on nuclear magnetic electromagnetic energy absorption of a fluid. The concept of transmission absorption with respect to a fluid is taught in addition to a system for guiding formation fluids from a pad into an assaying tubular section for sample analysis.
US 4 490 609 A discloses a method and apparatus for analyzing well fluids through irradiation by x-rays that aims to reduce the effects of the metal casing, cement and/or formation. Dual photon energy bands are used to independently measure the absorption from Thompson scattering and photoelectric effect. However, in the apparatus disclosed by Chevalier, measurements are made in the central section of the apparatus, thus the fluid must be displaced around the tool itself before measurement. Moreover, Chevalier does not teach of a method which does not disturb the fluid prior to or at the time of measurement, as the fluid would already have been displaced around the tool housing itself.
US 2 261 539 A describes a method and apparatus for analyzing well fluids through irradiation of said fluids by gamma rays from an isotope. Similar to Chevalier, the detected counts result from attenuation of the source photons in the annular fluids between the tool and the borehole wall. However, Egan does not teach a method wherein the fluid if not disturbed prior to or at the time of measurement, since the fluid would already have been displaced around the tool housing itself.
US 7 075 062 A describes a system and method for determining properties of a fluid based on x-ray irradiation of a borehole fluid and attenuation measurements. The concept of the link between Compton scattering measurements and the electron density is discussed as well as the possibility of using multiple energy bands. Chen also suggests a system for guiding formation fluids from a pad into an assaying section of the apparatus for sample analysis, and the concept of removal of spurious data resulting from sand influx into the system is also considered.
US 7 507 952 A describes a system and method for determining properties of a fluid based on the x-ray absorption of the fluid. The concept of transmission absorption with respect to a fluid is taught in addition to the concept of a fluid comparator cell.

There is, therefore, a longstanding need that remains unmet despite many prior unsuccessful attempts to achieve a forward-looking fluid analysis method that does not require transmission of a fluid sample from the wellbore into an apparatus, or otherwise disturbing the fluid.

In such a method, the radiation source and the imaging device would both need to be located within the tool housing near a low point of the apparatus, so that the fluid remains undisturbed and outside of the apparatus during measurement; in this manner, the various shortcomings of the prior art would be overcome. In addition, the prior art fails to teach of a mechanism through which an operator can anticipate fluid changes about to take place in front of the measuring tool prior to the tool reaching the interface; this approach would give an operator meaningful *a priori* knowledge regarding the status of the borehole, through the fluid composition of the borehole, in advance of the tool arriving at the sampled location.

The present inventors have now arrived at such a solution, example particulars of which are discussed below.

A method for identifying a fluid type inside a conduit is also provided, said method comprising: illuminating a fluid in a conduit using ionizing radiation; and detecting backscattered electromagnetic radiation returned from one or more surfaces of associated illumination volumes using one or more electromagnetic radiation sensors.

According to an example embodiment the method comprises: converting detected radiation into a corresponding set of planar image data. In a further example embodiment the method comprises: analyzing the image data using quantitative processing techniques. In a still further embodiment, the method further comprises: creating a depth-correlated log representative of the fluid response data. In a further embodiment still, the method comprises: converting detected radiation into a corresponding set of planar image data; analyzing the image data using quantitative processing techniques; and creating a depth-correlated log representative of the fluid response data.

According to another example embodiment, a system for identifying a fluid type inside a conduit is provided, said system comprising: means for illuminating a fluid in a conduit using ionizing radiation; and means for detecting backscattered electromagnetic radiation returned from one or more surfaces of associated illumination volumes using one or more electromagnetic radiation sensors.

In a further embodiment, the system comprises: means for converting detected radiation into a corresponding set of planar image data. In a still further embodiment, the system comprises: means for analyzing the image data using quantitative processing techniques. In a further embodiment still, the system comprises: means for creating a depth-correlated log representative of the fluid response data. In a yet further embodiment, the system comprises: means for converting detected radiation into a corresponding set of planar image data; means for analyzing the image data using quantitative processing techniques; and means for creating a depth-correlated log representative of the fluid response data.

A method for logging data relating to fluids within a conduit is provided, said method comprising: recording a plurality of fluid characteristic data signals obtained from a measurement tool while an apparatus in which the fluid is disposed remains stationary; comparing said obtained plurality of fluid characteristic data signals to a stored data bank of known fluid characteristic signals; and evaluating differences between said obtained plurality of fluid characteristic data signals and said stored data bank of known fluid characteristic signals in order to anticipate results from a subsequent fluid interrogation.

In a further embodiment, the method comprises: obtaining a plurality of data signal acquisitions obtained while longitudinally moving the apparatus a predetermined distance through the conduit between acquisition operations. In a still further embodiment, the method comprises: obtaining a plurality of data signal acquisitions obtained while longitudinally moving the apparatus a predetermined distance through the conduit during acquisition operations.

According to another example embodiment, a system for logging data relating to fluids within a conduit is provided, said system comprising: means for recording a plurality of fluid characteristic data signals obtained from a measurement tool while an apparatus in which the fluid is disposed remains stationary; means for comparing said obtained plurality of fluid characteristic data signals to a stored data bank of known fluid characteristic signals; and means for evaluating differences between said obtained plurality of fluid characteristic data signals and said stored data bank of known fluid characteristic signals in order to anticipate results from a subsequent fluid interrogation.

In a further embodiment, the system comprises: means for obtaining a plurality of data signal acquisitions obtained while longitudinally moving the apparatus a predetermined distance through the conduit between acquisition operations. In a further embodiment still, the system comprises: means for obtaining a plurality of data signal acquisitions obtained while longitudinally moving the apparatus a predetermined distance through the conduit during acquisition operations.

Preferred embodiments of the invention are described in the following with reference to the drawings. In the drawings:

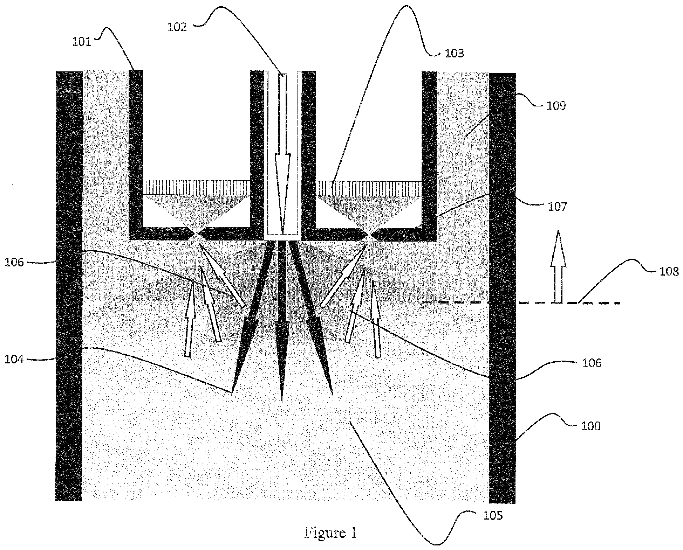

Figure 1 depicts an example embodiment comprising a tool disposed within a fluid-filled conduit containing an x-ray source, which is illuminating a volume of fluid separated by a fluid interface using x-rays; in this example, the scattered radiation resulting from the x-rays interaction with the fluid located in front of the tool is collected by a detector array or arrays;

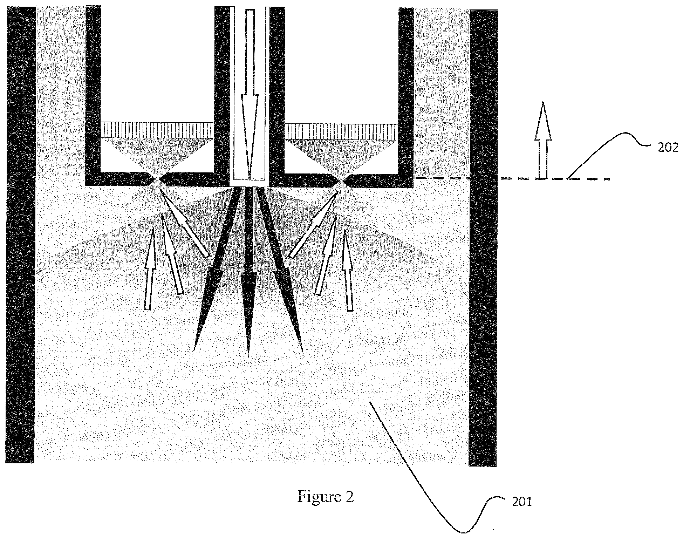

Figure 2 depicts the example embodiment of Figure 1, in which the tool has moved further into the conduit as illustrated by movement of the fluid interface into the fluid annulus between the tool and the conduit wall; consequently the scattering response of the fluid differs from the response of the interface between the two fluids in front of the tool;

There are no previously known technologies available on the market capable of providing an operator with non-disruptive means for determining the composition of a fluid or location of a fluid interface within a borehole with any significant level of detail with respect to the precise depth of a fluid interface or change.

In contrast, the methods and means described and claimed herein allow an operator to determine the precise depth location and characteristic of a fluid in a conduit through a forward-looking fluid analysis method that does not require removal of the fluid sample from the wellbore into an isolation apparatus. In this manner, the fluid remains undisturbed and outside of the apparatus during measurement, particularly in a region in front of the apparatus as it is lowered into the conduit.

The objects are achieved by acquiring radiation backscattered from fluids disposed in front of the tool in the area of the conduit that has not been disturbed by the action of the measurement. The backscattered radiation is collected by one or more detector arrays and analyzed in detail using computational comparative characterization techniques.

With reference now to accompanying Figures 1 and 2, in an example embodiment primary x-rays 104 are produced by an x-ray tube 102 located within a pressure resistance tool housing 101. The primary x-rays illuminate a section of the well fluids 105, 109 in front of the tool 101, which results in the backscattering of radiation from both the Thompson and Compton effects. The scattered radiation 106 enters a collimation device 107 such as a pinhole, optical slot, array collimator or other collimation means, and falls upon a detector array 103 or arrays. The scattered radiation 106 is distributed across the surface of the detector array 103, which in further embodiments comprises a linear array or an area array.

As compared to a detector based upon a single scintillator crystal and photomultiplier tube, a pixel array effectively comprises many individual detectors, and as the nature of a collimator will always reduce the number of incoming counts as compared to configurations lacking collimation, ordinarily skilled artisans will readily appreciate that a pixel array achieves a number of key benefits.

For example, by distributing the total collected number of counts over a number of pixels, the statistical noise associated with each pixel can be reduced when the all of the counts associated with all of the detectors are combined as a single reading. An idealized detector would be capable of producing noise statistics identical to Poissonian distribution; however, by increasing the number of individual detectors measuring an acquisition, it is possible to reduce the overall signal to noise ratio within acceptable standards when considering the short acquisition times required to capture a reasonable data rate when considering that the tool is moving through the fluid and therefore through the conduit.

Once all of the individual counts associated with each pixel of each detector have been summed, it can be assumed that the statistical noise has been reduced to such an extent that the useable signal to noise ratio is sufficient to determine changes in the overall acquisitioned count rate such that any appreciable change (*e.g.,* less than 1%) in fluid response would be detectable.

As the backscatter response of the fluid can be shown to be independent of the density of the fluid to the lowest order, it is possible to create a characteristic response of each of the fluid types an operator would expect to encounter in a fluid filled conduit.

According to further embodiments, the measured fluid response is then compared against a database of known fluid responses, and the specific fluid type determined as a function of the depth of the tool as it is moved through the conduit.

In a still further embodiment, the tool is held stationary and the fluid type is determined as a function of depth, using a combination of casing collar locators and run along depth of the wireline without requiring the tool to be moving through the conduit.

In a further embodiment still, the detector comprises a scintillator crystal coupled to a photo multiplier tube or photodiode. In yet another embodiment, the primary radiation 104 is produced by a chemical ionizing radiation source, such as a radioisotope.

In still further embodiments, the counts from each pixel of the detector array are not summed to obtain the total counts incident upon the entire detector, but instead individual pixels or groups of pixels are analyzed. This embodiment capitalizes on the highly localized region of space interrogated by each pixel in order to provide information about the spatial variations in fluid properties across the conduit. Furthermore, the scattered radiation recorded by different pixels or groups of pixels represents scattering through different angles as well as different attenuation path lengths. By comparing the signals received by different pixels with respect to these differences in scattering geometry, additional information can be obtained that may improve the fluid identification.

The foregoing specification is provided for illustrative purposes only, and is not intended to describe all possible aspects of the present invention. Moreover, while the invention has been shown and described in detail with respect to several exemplary embodiments, those of skill in the relevant arts will appreciate that minor changes to the description and various other modifications, omissions and additions may be made without departing from the scope thereof.

## Claims

1. A method for logging data relating to fluids (105, 109) within a conduit, said method comprising:
recording a plurality of fluid characteristic data signals obtained from a measurement tool (101) while an apparatus in which the fluid (105, 109) is disposed remains stationary;
comparing said obtained plurality of fluid characteristic data signals to a stored data bank of known fluid characteristic signals; and
evaluating differences between said obtained plurality of fluid characteristic data signals and said stored data bank of known fluid characteristic signals in order to anticipate results from a subsequent fluid interrogation.

2. The method of claim 1, further comprising: obtaining a plurality of data signal acquisitions obtained while longitudinally moving the apparatus a predetermined distance through the conduit between acquisition operations.

3. The method of claim 1 or 2, further comprising: obtaining a plurality of data signal acquisitions obtained while longitudinally moving the apparatus a predetermined distance through the conduit during acquisition operations.

4. A system for logging data relating to fluids (105, 109) within a conduit, said system comprising:
means for recording a plurality of fluid characteristic data signals obtained from a measurement tool (101) while an apparatus in which the fluid (105, 109) is disposed remains stationary;
means for comparing said obtained plurality of fluid characteristic data signals to a stored data bank of known fluid characteristic signals; and
means for evaluating differences between said obtained plurality of fluid characteristic data signals and said stored data bank of known fluid characteristic signals in order to anticipate results from a subsequent fluid interrogation.

5. The system of claim 4, further comprising: means for obtaining a plurality of data signal acquisitions obtained while longitudinally moving the apparatus a predetermined distance through the conduit between acquisition operations.

6. The system of claim 4 or 5, further comprising: means for obtaining a plurality of data signal acquisitions obtained while longitudinally moving the apparatus a predetermined distance through the conduit during acquisition operations.
